(19) Europäisches Patentamt / European Patent Office / Office européen des brevets

(11) **EP 4 316 469 B1**

(12) **EUROPEAN PATENT SPECIFICATION**

(45) Date of publication and mention
of the grant of the patent:
**28.01.2026  Bulletin 2026/05**

(21) Application number: **22774935.5**

(22) Date of filing: **01.03.2022**

(51) International Patent Classification (IPC):
*A61K 9/22* (2006.01)     *A61K 9/20* (2006.01)
*A61K 31/155* (2006.01)   *A61K 45/00* (2006.01)
*A61K 47/10* (2017.01)    *A61K 47/14* (2017.01)
*A61K 47/32* (2006.01)    *A61K 47/38* (2006.01)
*A61P 3/10* (2006.01)

(52) Cooperative Patent Classification (CPC):
**A61K 9/2027; A61K 9/2054; A61K 9/2095;
A61K 31/155; A61K 45/00; A61P 3/10**

(86) International application number:
**PCT/JP2022/008500**

(87) International publication number:
**WO 2022/202138 (29.09.2022 Gazette 2022/39)**

(54) **COMPOSITION FOR SUSTAINED-RELEASE SOLID DOSAGE FORM, SUSTAINED-RELEASE TABLET USING THE SAME, AND MANUFACTURING METHOD THEREOF**

ZUSAMMENSETZUNG FÜR EINE FESTE DARREICHUNGSFORM MIT VERZÖGERTER FREISETZUNG, TABLETTE MIT VERZÖGERTER FREISETZUNG, WELCHE DIESE VERWENDET, UND VERFAHREN ZUR HERSTELLUNG DERSELBEN

COMPOSITION POUR FORME POSOLOGIQUE SOLIDE À LIBÉRATION PROLONGÉE, COMPRIMÉ À LIBÉRATION PROLONGÉE L'UTILISANT ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **23.03.2021   JP 2021048020**

(43) Date of publication of application:
**07.02.2024   Bulletin 2024/06**

(73) Proprietor: **Mitsubishi Chemical Corporation
Chiyoda-ku
Tokyo 100-8251 (JP)**

(72) Inventors:
• **KOBAYASHI Ayaka
Tokyo 100-8251 (JP)**
• **YOSHIMURA Nobuyoshi
Tokyo 100-8251 (JP)**

(74) Representative: **Vossius & Partner
Patentanwälte Rechtsanwälte mbB
Siebertstrasse 3
81675 München (DE)**

(56) References cited:
**EP-A1- 3 674 379        WO-A1-2015/115453
CN-A- 110 833 537       JP-A- 2001 288 074
JP-A- 2014 224 086      JP-A- 2020 152 658
JP-A- 2020 531 649**

• YURI IKEUCHI-TAKAHASHI ET AL: "Formulation and Evaluation of Morin-Loaded Solid Lipid Nanoparticles", BIOLOGICAL & PHARMACEUTICAL BULLETIN, vol. 39, no. 9, 1 September 2016 (2016-09-01), JP, pages 1514 - 1522, XP055527640, ISSN: 0918-6158, DOI: 10.1248/bpb.b16-00300

Remarks:
The file contains technical information submitted after the application was filed and not included in this specification

**Description**

**TITLE OF THE INVENTION**

Composition for sustained-release solid dosage form, sustained-release tablet using the same, and manufacturing method thereof

**TECHNICAL FIELD**

The present invention relates to a composition for solid dosage form of medicament having a sustained-release property, a tablet as the solid dosage form medicament using the composition, and a method for manufacturing the same.

**BACKGROUND**

A sustained-release solid unit dosage form such as tablet can be controlled in release rate of a pharmaceutical ingredient so as to extend the release time of the pharmaceutical ingredient. Such a tablet may reduce frequency of administration and thereby reducing medication compliance, moreover, may lessen fluctuations in blood concentration and thereby lowering a side effect. Therefore, a sustained-release solid unit dosage has been studying and developing in recent years.

A tablet may contain additives such as excipient, binder, disintegrant, lubricant, and the like. The excipient does not have a physiological activity and may be added in an amount sufficient for assuring handleability during manufacturing operations. The binder may be added to bind particles of ingredient powder and may control the mechanical strength of a resultant tablet. The disintegrant, which can disintegrate the tablet by swelling due to absorption of water in the body, may be added so as to enhance the release of a pharmaceutical ingredient. The lubricant may be added so as to run the ingredient powder smoother and facilitate a pressing process for tableting.

A polyvinyl alcohol-based resin or PVA-based resin is water-soluble and biodegradable, and moreover is superior in adhesion to a wide variety of ingredients. Based on this property, PVA-based resin is commonly used as an additive for a pharmaceutical tablet, particularly as a binder, coating agent for film coated tablet etc. Among the above-mentioned additives, a binder and a disintegrant are known to influence not only the strength of a resulting tablet but also release rate of the pharmaceutical ingredient. Therefore, an application of polyvinyl alcohol-based resin to the preparation of a sustained-release solid dosage form has been investigated.

For example, JP 6657949 B (Patent Document 1) suggested a sustained-release pharmaceutical tablet. The sustained-release pharmaceutical tablet is imparted with a sustained-release property by containing a PVA fine particle having a gauche structure at least specific rate in the particle surface. The PVA-fine particle was used as a binder to enhance adhesiveness of ingredients in the tablet. In the Patent Document 1, a tablet was prepared by adding a PVA fine particle (pulverized product having a 50% particle diameter of 96 $\mu$m) having a saponification degree of 88% and an average polymerization degree of 2400 to a wet granulate blend of a pharmaceutical ingredient and crystalline cellulose, and subsequently mixing with crystalline cellulose, aerosil, and magnesium stearate to undergo compression molding. Thus produced tablet exhibited sustained-release as compared to a tablet containing a PVA fine particle (pulverized product having a 50% particle diameter of 100 $\mu$m) having a lower proportion of gauche structure (Fig. 5).

Further, JP 2020-152658 A (Patent Document 2) proposes a sustained-release solid dosage form containing a PVA having an average polymerization degree of 2000 or more and a saponification degree of 65 to 85 mol%. The patent document 2 discloses a tablet prepared by wet granulating process of a mixture of PVA, pharmaceutical ingredients, and crystalline cellulose, and undergoing compression molding of the granulates. The patent document 2 also discloses that the tablet containing a PVA having a saponification degree of 73.3 mol% or 80 mol% could be suppressed to less than half of a tablet containing a PVA having a saponification degree of 88 mol% in release rate after 6 hours. Moreover, the former tablet could reach 50% or less dissolution amount in release rate after 24 hours, which indicates a potential achievement of a sustained-release performance.

Furthermore, JP 2013-241341 A (Patent Document 3) discloses a sustained-release tablet, in which a modified PVA powder obtained by modifying polyvinyl alcohol (polymerization degree of 500 and 1700 and saponification degree of 88%) with acrylic acid and methyl methacrylate was contained as a matrix material. In the patent Document 3, the tablet was prepared by direct compression of a mixture of the matrix material (the modified PVA powder) with a crystalline cellulose as an excipient, and a pharmaceutical ingredient. The time period of reaching plateau in release rate of the tablet prepared by direct compression could be delayed as compared to a tablet using crystalline cellulose alone as a matrix materia Non-patent literature document Ikeuchi-Takahashi et al. (Non-Patent Document 1; Biol. Pharm. Bull. 39, 1514-1522, 2016) discloses morin loaded solid lipid nanoparticles.

**PRIOR ART**

## PATENT DOCUMENT

[Patent Document 1] JP 6657949 B
[Patent Document 2] JP 2020-152658 A
[Patent Document 3] JP 2013-241341 A

[Non-Patent Document 1] Biol. Pharm. Bull. 39, 1514-1522, 2016

## SUMMARY OF THE INVENTION

## TECHNICAL PROBLEM TO BE SOLVED BY THE INVENTION

By the way, a solid unit dosage form of medicament such as pharmaceutical tablet should comply with an established formulation standard for physical properties including hardness and friability. Therefore, a pharmaceutical excipient should have physical properties meeting with those determined in the formulation standard. The tablet disclosed in Patent Document 1 did not have a sufficient hardness satisfying the requirement, and is needed to increase the hardness for practical use as a sustained-release tablet.

According to the international pharmacopeias including Japan Pharmaceutical Excipients Standard (JPE), United States Pharmacopeia (USP) and European Pharmacopoeia (EP), a polyvinyl alcohol used for a tablet is required to have a saponification degree of 85 to 89 mol%. Therefore, the polyvinyl alcohol used in Patent Document 2 has a saponification degree outside of the required range.

According to Patent Document 3, a tablet with a hardness of 50N was obtained in Example by using a modified polyvinyl alcohol as a matrix. However, the pharmaceutical ingredient used in the Examples were nifedipine (evaluation 1 and evaluation 2) and carbazochrome sodium sulfonate (CCSS) (evaluation 3), which were poor or almost poor in water-solubility. A tablet containing such a poor or almost poor water-soluble ingredient has a tendency to take a longer duration, which looks like a sustained-release property. The Patent Document 3 fails to disclose that a tablet containing a readily water-soluble pharmaceutical ingredient can achieve a desired sustained-release property.

Under these situations, the purpose of the invention is to provide a composition for a sustained-release solid dosage form with a desired hardness and friability, using a polyvinyl alcohol satisfying the requirement of the international pharmacopeias for a pharmaceutical additive. In addition, a sustained-release tablet using the composition and a method for manufacturing the sustained-release tablet are also provided.

## MEANS FOR SOLVING THE PROBLEM

The inventors studied a combination of additives capable of providing a tablet with a satisfactory hardness and strength, in addition to a desired sustained-release property, using a polyvinyl alcohol-based resin meeting with requirements of Japan Pharmaceutical Excipients Standard (JPE), United States Pharmacopeia (USP), and European Pharmacopoeia (EP). As a result, the present invention was completed.

According to the present invention, a composition for sustained-release solid dosage form comprises (A) a polyvinyl alcohol-based resin having an average saponification degree of 78 to 96 mol% and an average polymerization degree of 800 or more, the polyvinyl alcohol-based resin (A) being a powder having an average particle size of 40 to 200 $\mu$m measured by a laser diffraction method; and (B) tannic acid as a polyhydric phenol compound.

The content of the (B) polyhydric phenol compound is preferably from 0.1 to 35 parts by weight with respect to 100 parts by weight of the polyvinyl alcohol-based resin (A).

A modification degree of the polyvinyl alcohol-based resin (A) is preferably less than 10 mol%. The polyvinyl alcohol-based resin (A) is in powder form having an average particle size of 40 to 200 $\mu$m.

The polyvinyl alcohol-based resin (A) may be a mixture of a first polyvinyl alcohol-based resin having an average polymerization degree of 1500 or more but not exceeding 3000 and a second polyvinyl alcohol-based resin having an average polymerization degree of 100 but less than 1500.

In one embodiment of the disclosure, a composition for sustained-release solid dosage form may further comprise (C) crystalline cellulose in an amount of 1 to 50 parts by weight with respect to 100 parts by weight of the polyvinyl alcohol-based resin (A).

The present invention also includes a method for manufacturing a sustained-release tablet of the disclosure. The manufacturing method comprises mixing tannic acid powder with a granule prepared from a composition comprising a pharmaceutical ingredient and a polyvinyl alcohol-based resin having an average saponification degree of 78 to 96 mol% and an average polymerization degree of 800 or more, and tableting. In a preferable embodiment, the polyvinyl alcohol-based resin has an average polymerization degree of 1500 to 3000 and is in powder form having an average particle size of 40 to 200 μm. The granule is preferably prepared by a fluid bed granulation process.

The average polymerization degree in this specification is referred as an average polymerization degree (number average polymerization degree) measured in accordance with JIS K 6726 (1994). In a tablet containing two or more types of polyvinyl alcohol-based resins with different average polymerization degree, the overall average polymerization degree corresponds to the sum of the individual number average polymerization degrees, calculated based on their content rates.

With respect to a tablet using a composition containing a polyvinyl alcohol-based resin and tannic acid, for example, JP 2014-224086 A (Patent Document 4) proposes an orally rapidly disintegrating solid dosage form containing a PVA modified with a polymerizable vinyl monomer (modified PVA) and tannic acid with a content ratio in weight of modified PVA : tannic acid = 1 : 0.1 to 10. As for the modified PVA, "POVACOAT type FM", which is a terpolymer of polyvinyl alcohol, acrylic acid and methyl methacrylate in a ratio of 32 : 1 : 7. Also, WO2015/115453 (Patent Document 5) discloses a tablet obtained by mixing excipients (D-mannitol, lactose hydrate) with polyvinyl alcohol (PE-05JPS, which has an average polymerization degree of 500), adding a tannic acid solution to the mixture, followed by granulating and tableting.

All tablets disclosed in these publications are rapidly disintegrating one. These documents taught that the disintegration time was prolonged in the absence of tannic acid. This means that tannic acid could shorten the disintegration time.

In addition, Patent Document 4 disclosed that a tablet with a higher hardness may be obtained in the absence of tannic acid. In other words, the presence of tannic acid may lower the hardness of a resultant tablet.

According to the invention, a composition for sustained-release solid dosage form may be improved in moldability and may be increased in tablet hardness with securing sustained-release of pharmaceutical ingredient. On the other hand, both Patent Documents 4 and 5 disclose a solid unit dosage form using a composition in which tannic acid is contained in combination with a polyvinyl alcohol-based resin, but the tannic acid is contained for imparting rapid disintegration property on the resultant tablet. Therefore, it is considered that the composition for solid dosage form of the invention and sustained-release tablet formulated from the composition are discriminated from the rapidly disintegrating tablet disclosed in Patent Documents 4 or 5 in chemical states of the polyvinyl alcohol-based resin and the tannic acid in the tablet.

## EFFECT OF THE INVENTION

A composition for sustained-release solid dosage form of the disclosure can attain a hydrogelation through hydrogen bonding between the polyvinyl alcohol-based resin and the tannic acid contained therein. Thus formed hydrogel can prolong the duration of the pharmaceutical ingredient contained in the solid dosage form regardless of water-solubility of the pharmaceutical ingredient. In addition, the composition for sustained-release solid dosage form may have an excellent compressibility and provide a sustained-release tablet with a relatively high hardness.

## EMBODIMENT FOR CARRYING OUT THE INVENTION

[Composition for sustained-release solid dosage form]

(A) Polyvinyl alcohol-based resin used as a matrix material

A polyvinyl alcohol (PVA)-based resin used in the composition for solid dosage form of the present invention is a resin mainly composed of vinyl alcohol structural units obtained by saponifying a polyvinyl ester-based resin which is a polymer of a vinyl ester-based monomer. The PVA-based resin contains a vinyl ester structural unit as an unsaponified portion, in addition to the vinyl alcohol structural unit in an amount corresponding to the saponification degree.

The polyvinyl alcohol (PVA)-based resin used in the composition for solid dosage form is not limited to an unmodified PVA-based resin composed of a vinyl alcohol unit and a vinyl ester unit. A modified polyvinyl alcohol-based resin or a post-modified PVA-based resin may be used. A modified polyvinyl alcohol-based resin is a saponified copolymer of vinyl ester-based monomer and copolymerizable monomer with the vinyl ester-based monomer. In the case of the modified PVA-based resin, the modification degree, which is correspondent to a content of structural units other than a vinyl alcohol unit and a vinyl ester structural unit, is less than 10 mol%, preferably 5 mol% or less, and particularly preferably 1 mol% or less.

Examples of the vinyl ester monomer include vinyl formate, vinyl acetate, vinyl propionate, vinyl valerate, vinyl butyrate, vinyl isobutyrate, vinyl pivalate, vinyl caprate, vinyl laurate, vinyl stearate, vinyl benzoate, vinyl versatate and the like. Of these vinyl esters, vinyl acetate is preferably used for a practical reason.

Examples of the copolymerizable monomer include olefins such as ethylene, propylene, isobutylene, a-octene, α-dodecene, and α-octadecene; hydroxy group-containing α-olefins such as 3-buten-1-ol, 4-penten-1-ol, 5-hexen-1-ol, 3,4-dihydroxy-1-butene and their derivatives such as acylated products; unsaturated acids such as acrylic acid, methacrylic acid, crotonic acid, maleic acid, maleic anhydride, itaconic acid, undecylenic acid, and salts, monoesters, or dialkyl esters thereof; nitriles such as acrylonitrile and meta-acrylonitrile; amides such as diacetoneacrylamide acrylamide and methacrylamide; olefin sulfonic acids such as ethylene sulfonic acid, allyl sulfonic acid, methallyl sulfonic acid or salts thereof; alkyl vinyl ethers; vinyl compounds such as dimethylallyl vinyl ketone, N-vinylpyrrolidone, vinyl chloride, vinylethylene carbonate, 2,2-dialkyl-4-vinyl-1,3-dioxolane, and glycerin monoallyl ether; substituted vinyl acetates such as isopropenyl acetate and 1-methoxyvinyl acetate, vinylidene chloride, 1,4-diacetoxy-2-butene, 1,4-dihydroxy-2-butene, vinylene carbonate and the like. Such copolymerizable monomers may be used alone or in combination of two or more of them. An amount of the copolymerizable monomer is less than 10 mol%, preferably 5 mol% or less, particularly preferably 1 mol% or less, based on the total amount of monomers contained in the polymer.

A preferable modified PVA-based resin is a copolymer in which an unsaturated acid, or salt, monoester, or dialkyl ester thereof is copolymerized as the copolymerizable monomer. A polyvinyl alcohol/acrylic acid/methyl methacrylate copolymer is particularly preferred as a modified PVA-based resin.

The vinyl ester-based monomer (optionally including a copolymerizable monomer) can be polymerized by a conventionally known method (bulk polymerization, solution polymerization, suspension polymerization, dispersion polymerization, emulsion polymerization, etc.). As a polymerization catalyst and a solvent used in the polymerization, a conventionally known one may also be used. In general, solution polymerization using a methanol solvent may be applied.

Saponification of the resulting vinyl ester-based polymer can also be carried out by a conventionally known method. Industrially, the vinyl ester polymer is dissolved in alcohol and saponified in the presence of an alkali catalyst. A common alkali catalyst includes hydroxides of alkali metals such as sodium hydroxide, potassium hydroxide and sodium methylate, and alcoholates.

The polyvinyl alcohol-based resin particles obtained by saponification are washed with a lower alcohol such as methanol, and then dried with hot air or the like in a continuous or batch manner to obtain PVA-based resin dry powder. An appropriate drying temperature is commonly from 50 to 150°C, preferably 60 to 130°C, particularly preferably 70 to 110°C. An unduly high drying temperature may cause a thermal deterioration of a PVA-based resin particle. An unduly low drying temperature extends a drying time. An appropriate drying time is commonly for 1 to 48 hours, preferably 2 to 36 hours. If PVA-based resin particles are subjected to drying operation for an unduly long period, the PVA-based resin particles tend to be thermally deteriorated. If PVA-based resin particles are subject to drying operation for unduly short period, insufficiently dried PVA-based resin particles may be obtained or the drying operation need to conduct at a higher drying temperature. A PVA-based resin is dried up to usually 10% by weight or less, preferably 5% by weight or less, and particularly preferably 1% by weight or less, in the content of the solvent.

The average saponification degree of the PVA-based resin used in the disclosure is in the range of 78 to 96 mol%, and preferably 85 to 89 mol%. This range meets with the saponification degree of a partially saponified polyvinyl alcohol specified in the international standard including Japan Pharmaceutical Excipients Standard (JPE), the European Pharmacopoeia (EP) and the United States Pharmacopoeia (USP).

PVA-based resin having an average saponification degree within the said **range may exhibit** high water solubility, and after oral administration, hydrogen bonds between hydroxyl groups of a polyhydric phenol compound and crystalline cellulose may be formed to result in gelation. The gel may aid sustained-release of pharmaceutical ingredients. On the other hand, PVA-based resin having unduly low saponification degree has a tendency to reduce gelation due to reduced hydroxyl groups, resulting in lack of contribution to sustained-release. Poor gel-forming ability may also decrease the strength of the resin itself, and moreover lower tablet hardness. The average saponification degree herein refers to the saponification degree measured in accordance with JIS K 6726 (1994).

The average polymerization degree of the PVA-based resin used in the disclosure is 800 or more, more preferably 1500 or more to 3000, still more preferably 2100 to 2600. A PVA-based resin having too low average polymerization degree may not sufficiently suppress the release of the pharmaceutical ingredient due to poor gel-forming ability. A common polyvinyl alcohol-based resin used as a binder has an average polymerization degree of less than 800. Such a PVA-based resin having a relatively low polymerization degree is likely to dissolve in water rather than gelation after oral administration. This means that the release of the pharmaceutical ingredient may not be sufficiently suppressed for assuring a desirable sustained-release. On the other hand, powder of PVA-based resin having unduly high average polymerization degree may impair compressibility of the PVA-based resin powder due to an increase of elasticity, as a result, tableting moldability is lowered. For these reasons, a preferable average polymerization degree of the PVA-based resin is 3000 or less. The average polymerization degree herein refers to an average polymerization degree (number average polymerization degree), which is measured in accordance with JIS K 6726 (1994).

In addition, the viscosity at 20°C of a PVA-based resin aqueous solution having a concentration of 4% by weight is preferably 6.0 mPa • s or more, more preferably 15.0 to 70.0 mPa • s, and still more preferably 20.0 to 60.0 mPa • s. The aqueous solution (4 % by weight) of PVA-based resin having an unduly high viscosity is usually caused from unduly high average polymerization degree. Therefore, a PVA-based resin having such unduly high viscosity with respect to the PVA-based resin aqueous solution (4% by weight) may be impaired in compressibility and tableting moldability of the PVA-based resin powder due to an increase of elasticity. On the other hand, unduly low viscosity of a PVA-based resin aqueous solution having a concentration of 4% by weight is caused from unduly low average polymerization degree. Therefore, a PVA-based resin having such unduly low viscosity with respect to the PVA-based resin aqueous solution (4% by weight) could not impart a desired sustained-release property on a resultant tablet. In this specification, the viscosity at 20°C of a PVA-based resin aqueous solution having a concentration of 4% by weight refers to a viscosity measured in accordance with JIS K 6726 (1994).

The polyvinyl alcohol-based resin (A) in powder form may be mixed with ingredients. Alternatively, a granule of the PVA-based resin prepared from its aqueous solution may be directly mixed with other ingredients. In the case of using PVA-based resin as a matrix material, a polyvinyl alcohol-based resin (A) in powder form is preferred from the viewpoint of productivity.

In the case of using a PVA-based resin powder, the average particle size is in the range of 40 $\mu$m to 200 $\mu$m, preferably 45 to 100 $\mu$m, more preferably 45 to 80 $\mu$m, further preferably 45 to 70 $\mu$m. A PVA-based resin having unduly large average particle size has a tendency to provide a tablet with a relatively low hardness. The average particle diameter herein refers to a particle diameter measured by a laser diffraction method.

A polyvinyl alcohol-based resin (A) having above-mentioned desirable properties is suitable for a matrix material of a sustained-release solid dosage form. When the polyvinyl alcohol-based resin (A) is in contact with water in the presence of a compound having a lot of hydroxyl groups such as a polyhydric phenol compound described later, the polyvinyl alcohol-based resin (A) is able to form a three-dimensional network structure through a hydrogen bond with the compound to result in gelation. It is considered that this gelation may aid to suppress the release of the pharmaceutical ingredient dispersedly contained in the matrix, thereby exhibiting a sustained-release property. The higher the polymerization degree of the PVA-based resin, the more complicated three-dimensional network structure may be formed due to an increased entanglement of the PVA-based resin with tannic acid. Therefore, the PVA-based resin having a higher polymerization degree would enhance the sustained-release property.

(B) Polyhydric phenol compound

Examples of the polyhydric phenol compound (B) used in the disclosure include catechol, resorcinol, hydroquinone, pyrogallol, oxyhydroquinone, phloroglucin, tannic acid and the like. These may be used alone or in combination of two or more. Among these polyhydric phenols, tannic acid is claimed.

Tannic acid can be extracted from a variety of plant materials. For example, tannic acid can be extracted with water or ethanol from persimmon fruit, chestnut astringent skin, quincunx, gallnut, cod powder, leguminous tamarind seed coat, or mimosa bark. A preferable tannic acid is a tannic acid extracted from quincunx or gallnut which is listed in the 17th revision of the Japanese Pharmacopoeia. Either unrefined or refined tannic acid may be used, but refined tannic acid is preferably used.

Tannic acid is preferably contained in an amount of 0.1 to 35 parts by weight with respect to 100 parts by weight of PVA-based resin (A) as a matirx, that is to say, the preferable content ratio of tannic acid to the matrix PVA is from 0.0001 to 0.35. The amount of the tannic acid contained with respect to 100 parts by weight of matrix PVA-based resin (A) is preferably from 1 to 25 parts by weight, more preferably from 5 to 15 parts by weight. If the amount of tannic acid is too high, a resultant tablet tends to be colored.

Polyhydric phenol compounds such as tannic acid have a high affinity for water, and can form a hydrogen bond with a hydroxyl group of PVA-based resin when PVA-based resin is present. The hydrogen bond between them can promote the formation of a three-dimensional network structure in a tablet. A gel having a three-dimensional network structure can suppress the release of pharmaceutical ingredients contained in the tablet, and as a result, sustained-release can be achieved.

Incidentally, both Patent Documents 4 and 5 disclose an orally rapidly disintegrating tablet containing polyvinyl alcohol and tannic acid. In the disclosure of these patent documents, a PVA having a relatively low polymerization degree and low gel-forming ability was used, and granules prepared from a solution containing the PVA and tannic acid were compressed to produce a tablet. This combination could not form an intended three-dimensional network structure. The tannic acid, which has a high affinity for water, is dispersed all over the tablet. For this condition, the tannic acid may attract water around the surface of the tablet, and carry the water towards the inside of the tablet like a water channel. This action may be considered as a rapid disintegration property. Thus, the function of tannic acid in the tablet disclosed in the Patent Document 4 or 5 is considered as distinguishable from the function of tannic acid in a composition for sustained-release solid dosage form of this disclosure.

Since polyhydric phenol (B) such as tannic acid is in powder form at room temperature, the polyhydric phenol (B) may be used as it is. However, a granule prepared from a polyhydric phenol solution by spraying and being dried may also be used.

The particle size of tannic acid powder is usually from 5 to 100 $\mu$m, preferably 10 to 50 $\mu$m.

(C) Crystalline cellulose

Crystalline cellulose is contained as an excipient. Since crystalline cellulose is excellent in compressibility, tablet hardness can be increased by containing such crystalline cellulose.

For the crystalline cellulose, a crystalline cellulose powder having an average particle size of less than 150 μm, in particular, from 100 to 140 μm is preferably used, but not limited thereto.

The content of crystalline cellulose with respect to PVA-based resin is in a ratio in weight of 100/1 to 100/50, preferably 100/5 to 100/35, more preferably 100/10 to 100/30, in terms of PVA-based resin/crystalline cellulose.

(D) Other additives

The composition for a solid dosage form of the disclosure may contain additives other than the said ingredients within a range that does not impair the effects of the disclosure, for example, 20% by weight or less, preferably 10% by weight or less of the composition. Examples of other additives include excipient, binder, disintegrant, pH adjuster, fluidizing agent, surfactant, coloring agent, sweetener and coating agent.

(D-1) Binder

A binder may be added to promote bindings of particles in the process of dry or wet granulating, and tableting by direct compression or wet process. Such binders are preferably used to control the disintegration of solid unit dosage form including a tablet.

As the binder, dextrin, gum arabic, gelatin, hydroxypropyl starch, methyl cellulose, hydroxypropyl cellulose, hypromellose, pullulan, starch paste, polyvinyl alcohol-based resin and the like may be used.

The amount of the binder based on 100 parts by weight of the composition for granulation, is usually from 0.1 to 10 parts by weight, preferably 0.2 to 5 parts by weight, more preferably 0.5 to 3 parts by weight, depending on the type of binder compound used.

A polyvinyl alcohol-based resin used as the binder is distinguishable from the polyvinyl alcohol-based resin used as a matrix material which is the component (A), because of different average polymerization degrees. The average polymerization degree of the polyvinyl alcohol-based resin used as the binder is in the range of preferably 100 to less than 1500, more preferably 200 to 1000. A binder PVA-based resin usually has an average saponification degree of 70 to 99 mol%. However, a PVA-based resin having an average saponification degree of 78 to 96 mol%, preferably 85 to 89 mol%, which is the same range as that of a matrix PVA-based resin, may be normally used as a binder.

Similar to the polyvinyl alcohol-based resin used for the matrix material, a modified polyvinyl alcohol-based resin or saponified copolymer modified with a copolymerizable monomer described earlier may also be used as a binder.

A polyvinyl alcohol-based resin in a form of aqueous solution is preferably used as a binder.

When a said polyvinyl alcohol-based resin is employed as a binder, a resulting tablet contains two or more types of polyvinyl alcohol-based resin differing in average polymerization degree. In the case that a tablet contains two or more types of polyvinyl alcohol-based resins differing in average polymerization degree, the overall average polymerization degree of the polyvinyl alcohol-based resins contained in the composition for solid dosage form is employed. The overall average polymerization degree corresponds to the sum of the individual average polymerization degrees calculated based on their content rates.

For example, in the case of containing two types of PVA-based resins, i.e. a matrix PVA-based resin and a binder PVA-based resin, the average saponification degree and average polymerization degree may be calculated respectively by the following formulas, wherein $SD_m$ and $P_m$ are referred as a saponification degree and polymerization degree of matrix PVA-based resin respectively, $SD_b$ and $P_b$ are referred as a saponification degree and polymerization degree of a binder PVA-based resin respectively, and M and B are the respective content rates of matrix PVA-based resin and binder PVA-based resin to the total amount of PVA-based resins. Note that M+B=1.

$$\text{Average saponification degree} = SD_m \times M + SD_b \times B$$

$$\text{Average polymerization degree} = P_m \times M + P_b \times B$$

The content of the PVA-based resin used for the binder (B) is typically at most about 5% by weight (usually 3% by weight or less, preferably 2.5% by weight or less) of the total PVA-based resin. Therefore, even if the binder PVA-based resin having an average polymerization degree less than 800 is contained, the measurement value of the average polymerization degree of the PVA-based resin contained in the composition for solid dosage form may be 800 or more.

(D-2) Lubricant

A lubricant may be added so as to improve fluidity, provide lubricity during compression in the die and during tablet ejection, and avoid sticking to the punch faces or die wall. Examples of the lubricant include stearic acid, magnesium stearate, calcium stearate, talc, anhydrous silicic acid, and the like.

(D-3) Excipient

An excipient other than the said crystalline cellulose, which may act as a bulking agent, may be used alone or together with crystalline cellulose.

Excipients that can be used include sugar alcohols (such as mannitol, erythritol, xylitol, sorbitol and maltitol), sugars (such as glucose, fructose, lactose, sucrose, trehalose, maltose, and oligosaccharides), calcium phosphates, starches, sodium phosphates and gelatin. Among these, a sugar alcohol, in particular, mannitol is preferably used.

(D-4) Others

In addition to the above-listed additives, disintegrants (e.g., carmellose calcium, carboxymethyl starch sodium, croscarmellose sodium, crospovidone, cellulose or derivatives thereof, and starch or derivatives thereof); pH adjusters (e.g., citric acid and its salts, phosphoric acid and its salts, carbonic acid and its salts, tartaric acid and its salts, fumaric acid and its salts, acetic acid and its salts, amino acids and its salts, succinic acid and its salts and lactic acid and its salt); flow agents (e.g., light anhydrous silicic acid, hydrated silicon dioxide, titanium oxide, stearic acid, corn gel, and heavy silicic anhydride); surfactants (e.g., phospholipids, glycerin fatty acid esters, polyoxyethylene fatty acid esters, sorbitan fatty acid esters, polyethylene glycol fatty acid esters, polyoxyethylene hydrogenated castor oil, polyoxyethylene alkyl ethers, sucrose fatty acid esters, sodium lauryl sulfate, polysorbates, sodium hydrogen phosphates and potassium hydrogen phosphates); coloring agents (e.g., iron sesquioxide, yellow iron sesquioxide, food yellow No. 5, food yellow No. 4, aluminum chelate, titanium oxide, and talc); and sweeteners (e.g., saccharin, aspartame, acesulfame potassium, thaumatin and sucralose) may be contained.

[Active pharmaceutical ingredient (API)]

Examples of the active pharmaceutical ingredient applicable to the solid dosage form of the disclosure include antipyretic analgesic antiphlogistics, nutrient and tonic supplements, psychotropics, antidepressants, antianxiety drugs, hypnosedatives, anticonvulsants, CNS-acting drugs, brain metabolism improving agents, brain circulation improving agents, antiepileptic agents, sympathomimetic drugs, gastrointestinal drugs, acid suppressants, anti-ulcerogenic drugs, cough medicines, antiemetics, anapnoics, bronchodilators, allergic drugs, antihistamine agents, agents for dental administration or oral administration, cardiants, agents for cardiac arrhythmia, diuretics, hypertension drugs, vasoconstrictors, coronary vasodilators, peripheral vasodilators, blood coagulation inhibitors, hyperlipidemias agents, cholagogues, antibiotics, chemotherapeutic agents, diabetes drugs, osteoporosis drugs, antirheumatics, skeletal muscle relaxants, antispasmodics, hormonal agents, alkaloid drugs, sulfa drugs, arthrifuges, and antineoplastics.

Since the composition for solid dosage form of the disclosure can impart a sustained-release property, it is preferably used in combination with a pharmaceutical ingredient that requires sustained-release property, or a readily water-soluble pharmaceutical ingredient. In particular, oral dosage form containing a readily water-soluble pharmaceutical ingredient as the active pharmaceutical ingredient requires a sustained-release property.

In this specification, readily water-soluble pharmaceutical ingredients are drugs classified as very soluble, freely soluble, or soluble in dissolution in water indicated in the Japanese Pharmacopoeia 18th edition, or Class I or III in the BCS (Biopharmaceutical Classification System).

Examples of readily water-soluble pharmaceutical ingredients include metformin hydrochloride, sodium salicylate, aminopyrine, benzydamine hydrochloride, benzydamine hydrochloride, tiaramide hydrochloride, doxycycline hydrochloride n-hydrate, moxalactam sodium, bacampicillin hydrochloride, levamisole, dl-methylephedrine hydrochloride, noscapine, codeine phosphate hydrate, dihydrocodeine phosphate, cloperastine hydrochloride, dl-isoprenaline hydrochloride, L-ethylcysteine hydrochloride, L-methylcysteine hydrochloride, tolperisone hydrochloride, eperisone hydrochloride, tizanidine hydrochloride, roxatidine acetate hydrochloride, chlorpheniramine maleate, diphenhydramine hydrochloride, imipramine hydrochloride, clomipramine hydrochloride, diphenhydramine hydrochloride, chlorpheniramine maleate, clemizole hydrochloride, methoxyphenamine hydrochloride, etilefrine hydrochloride, alprenolol hydrochloride, bufetrol hydrochloride, oxprenolol hydrochloride, procainamide hydrochloride, disopyramide phosphate, mexiletine hydrochloride, diltiazem hydrochloride, isosorbide mononitrate, diltiazem hydrochloride, dilazep hydrochloride hydrate, hexamethonium bromide, clonidine hydrochloride, diltiazem hydrochloride, buformin hydrochloride, isoniazid, ethambutol hydrochloride, thiamazole, vitamin C, B1, or B2, loxoprofen Na, oxybutynin hydrochloride and the like.

The composition for solid dosage form of the disclosure can provide a tablet with a desirable moldability, hardness, and strength while assuring sustained-release property. In this connection, the composition for solid dosage form may provide a sustained-release oral tablet having a desired hardness and strength, even when used in combination with a pharmaceutical ingredient commonly recognized as poor compressibility. Therefore, the disclosed composition is particularly useful.

The active pharmaceutical ingredient (API) as exemplified above may be contained at a ratio in weight of the matrix PVA-based resin (A) to API, matrix PVA-based resin (A) : API, ranging from 0.5/100 to 1000/100, preferably from 20:100 to 500:100, more preferably from 30:100 to 300:100.

In addition, the content ratio in weight of the polyhydric phenol (B) to API, polyhydric phenol (B) : API, is preferably from 0.05:100 to 30:100, more preferably from 0.1:100 to 20:100, particularly preferably from 1:100 to 10:100. When a pharmaceutical ingredient with poor moldability is contained as the API, and the content of polyhydric phenol (B) which contributes to increase in hardness is too low, a resultant tablet would not have satisfactory hardness and would not exhibit satisfactory sustained-release property either.

<Sustained-release tablet and its manufacturing method thereof>

A sustained-release tablet of the invention is a tablet containing a pharmaceutical ingredient and a disclosed composition for sustained-release solid dosage form.

The term "sustained-release tablet" as used herein means that a tablet controlled in release rate, release time, and delivered site of the active pharmaceutical ingredient from the tablet with a formulation in accordance with the Japanese Pharmacopoeia 18th edition for the purpose of reducing the frequency of administration or reducing a side effect.

The kind of pharmaceutical ingredient to be contained is not particularly limited, but a sustained-release property-required ingredient or a readily water-soluble ingredient is preferably used as the pharmaceutical ingredient.

A tablet is commonly produced by blending ingredients to be contained and thereafter either tableting the blend through directly compression or tableting by pressing granules prepared from the blend. A sustained-release tablet of the disclosure is featured by uniformity dispersion of a pharmaceutical ingredient contained in the matrix PVA-based resin (A) used in the tablet. Such a tablet may be obtained by uniformly dispersing the pharmaceutical ingredient in the matrix PVA-based resin (A), and thereby confining the pharmaceutical ingredient in a hydrogel formed from the matrix PVA-based resin (A). The tablet may have a desired sustained-release of the pharmaceutical ingredient.

Since a pharmaceutical ingredient is confined in a matrix gel (hydrogel) formed through hydrogen bonds, even a tablet containing a pharmaceutical ingredient classified as readily water-soluble one in dissolution can exhibit sustained-release performance.

A conventional method for tableting by compression molding may be employed as long as the method may provide a tablet in which the pharmaceutical ingredient is homogeneously dispersed in the matrix PVA-based resin.

For specifically exemplified tableting methods, a tableting by compression such as a direct powder compression (direct compression), a semi-dry granules compression, granules compression, or a tableting by wet molding process may be used.

The direct powder compression is a method of directly mixing a pharmaceutical ingredient and an additive, and pressing the mixture in the absence of water. The dry granules compression is a method of granulating by dry process (or slugging) and then tableting by compression. The dry granules compression may achieve higher uniformity in the resultant tablet than the direct powder compression. Each of the semi-dry granule compression and the semi-direct compression is a method of preparing a granule containing only additives but not containing a pharmaceutical ingredient by wet process and thereafter adding the pharmaceutical ingredient, followed by tableting. The use of granules prepared by wet process may enhance a cohesive strength in a resulting tablet, which can prevent powdering of the surface of the tablet. The wet granule compression is a method of obtaining a granule containing a pharmaceutical ingredient and additives by wet process and then tableting the granules. The tableting by wet molding process is a method of molding or punching out the wet blend into a desirable shape, and then drying removing solvents such as water from the shaped product.

Of the above-mentioned production methods, tableting by compression is preferred. The tableting by compression may be conducted by preparing a granule containing the matrix PVA-based resin (A) and the pharmaceutical ingredient in advance, and then mixing the granules with polyhydric phenol (B), followed by tableting via compression.

A mixture (composition for preparing granule) containing an excipient ((C) crystalline cellulose) in addition to the matrix PVA-based resin (A) and the pharmaceutical ingredient may be applied to the granulation process.

A binder may optionally be added to the composition for granulation. Moreover, other additives may optionally be added to the composition for granulation.

Examples of the granulation process include a wet granulation, dry granulation and spray-dry granulation. The wet granulation includes a granulation process of the mixture together with a binder solution, and a granulation process of wet lump obtained by kneading the mixture. The dry granulation is a granulation process comprising milling a product by compression molding of dry powder. The spray-dry granulation is a granulation process comprising preparing a slurry of powder with use of a large amount of water, spraying the slurry and drying to form granules.

The solvent in the binder solution is appropriately selected according to the type of compound used as the binder. In the case of a binder PVA, water can be used as a solvent.

As a granulator, a dry granulator, a basket-type extrusion granulator, a stirring granulator, a centrifugal tumbling granulator, a fluid bed granulator, spray drying granulator and the like may be used. The dry granulator is a machine for performing a dry granulation by applying a high pressure to a powder blend to create a lump, breaking down the lump to obtain powder having an appropriate particle size. The stirring granulator is a machine for granulation by mixing the powder ingredients with a stirring blade for several minutes, and adding a liquid binder solution dropwise to perform granulation during stirring. The fluid bed granulator is a machine for granulation which comprises mixing powder ingredients fluidized with air to form a powder blend and spraying a liquid binder with a nozzle in a convection or countercurrent flow to help the powder blend for agglomeration by spray droplets, and then drying to enlarge the size of the agglomerate. The spray drying granulator is a machine for performing granulation by spray drying. A granulation process using a fluid bed granulator is favored from the viewpoint of a balance between productivity and facilitation in the production of granules having excellent compressibility.

In a typical wet granulation, ingredients are kneaded with a solvent and then granulated. Examples of this wet granulation include a granulation by milling, granulation by extrusion, granulation by stirring, granulation by tumbling process, and the like. The wet granulation may use alcohols such as ethanol and isopropanol, or water as the solvent.

Subsequently, thus prepared granules are pressed to form a tablet. The polyhydric phenol (B) is preferably mixed with the granules during the tableting process. In order to improve compressibility, a lubricant is preferably mixed in addition to the polyhydric phenol (B).

A tableting by compression may be performed with use of a conventional machine known in the pharmaceutical field, such as rotary tableting machine, a single-shot tableting machine, or the like. For example, a tablet may be formed by a direct powder compression in which ingredients to be contained are evenly mixed and then compressed. A wet granule compression or a dry granule compression in which granules prepared from ingredients by wet granulation or dry granulation are pressed to form a tablet may be also applied.

Among these tableting processes, the wet granule compression is preferred because of smoother fluidity and higher uniformity during the mixing process.

A tableting pressure, which is a pressure applied in compression molding, is preferably about 1 kN or more, more preferably about 2 kN or more, and even more preferably about 4 kN or more, but preferably about 60 kN or less, more preferably about 50 kN or less, and even more preferably about 30 kN or less. This range of tableting pressure may not put excessive strain the punch used for tableting process, it is possible to maintain a consistent level of pressure throughout the compression process.

The pharmaceutical tablet may be ellipsoidal, cylindrical, spherical, doughnut-shaped, or any other shaped. Moreover, the tablet may be coated with a film, according to necessity.

The sustained-release tablet manufactured by above-mentioned processes normally has a hardness of 50 to 200 N, depending on a shape or size of the tablet, and may satisfy the properties required as a tablet. The sustained-release tablet with a diameter of 11 mm has a hardness of 80 to 150 N, preferably 90 to 130 N.

The sustained-release property depends on a formulation of the tablet, especially average polymerization degree and average saponification degree of polyvinyl alcohol-based resin, type of polyhydric phenol compound, content ratio of polyvinyl alcohol-based resin and polyhydric phenol compound, type and content ratio of the pharmaceutical ingredient, and so on. However, the sustained-release tablet manufactured by above-mentioned processes may exhibit sustained-release regardless of the level of water-solubility of a pharmaceutical ingredient contained therein.

Regarding the release behavior in accordance with a dissolution test based on DISSOLUTION Test 1 of USP41 "Metformin Hydrochloride Extended-Release Tablets", the released amount of pharmaceutical ingredient from the sustained-release tablet of the disclosure can fall in the following ranges. The dissolution test was conducted by dissolving 500 mg of the sustained-release tablet at 37°C in 900 mL of distilled water, with use of a dissolution tester NTR-6400ACT (manufactured by Toyama Sangyo Co., Ltd.). The release rate of pharmaceutical ingredient from the tablet after 1 hour and 3 hours from the start of measurement was calculated on the basis of the dissolution rate after 10 hours as 100%. The dissolution rate reached plateau or almost steady state after 10 hours from the start of measurement, which can be assumed as 100% of the dissolution rate.

Release rate of pharmaceutical ingredient after 1 hour from the start of measurement: 10 to 45%, preferably 20 to 43%, more preferably 30 to 42%.

Release rate of pharmaceutical ingredient after 3 hours from the start of measurement: 45 to 75%, preferably 50 to 73%, more preferably 55 to 70%.

EP 4 316 469 B1

**EXAMPLES**

The present invention will be further described below with reference to examples and comparative examples, but the present invention is not limited to the following examples.

[Measurement and evaluation method]

(1) Tablet hardness (N)

A hardness of a tablet manufactured by compression was measured using a PC-30 hardness tester (manufactured by OKADA SEIKO.CO., LTD.).

(2) Release rate

Dissolution Test 1 of USP41 "Metformin Hydrochloride Extended-Release Tablets" was conducted on a tablet containing metformin which is a readily water-soluble pharmaceutical ingredient.
The dissolution test was conducted by dissolving 500 mg of tablets in 900 mL of distilled water at 37°C and measuring the amount of the pharmaceutical ingredient (metformin) with a dissolution tester NTR-6400ACT (manufactured by Toyama Sangyo Co., Ltd.) after 1 hour, 3 hours, and 10 hours from the start of the test. The release rate (%) after 1 hour and 3 hours was calculated when the dissolution amount after 10 hours was taken as 100%.

[Manufacturing of tablets]

A PVA-based resin for a matrix material, metformin hydrochloride as a pharmaceutical ingredient, and crystalline cellulose ("PH102" manufactured by Asahi Kasei Chemicals) were mixed at a ratio shown in Table 1. This mixture was fed to a granulator ("MP-01" manufactured by Powrex Corporation) and fluidized. A PVA aqueous solution having a concentration of 6%, which was used as a liquid binder, was also fed to the granulator and produce granules. The PVA aqueous solution (6%) was prepared by dissolving 1.0 part of a PVA-based resin (average polymerization degree of 600 and saponification degree of 88 mol%) in water and had a viscosity 5.0 mPa·s in the case of 4% aqueous solution. The conditions for granulation process were as follows: supply air temperature 70°C, supply air volume 0.9 m$^3$/min, atomizing air pressure 0.5 MPa, and atomizing air volume 30 NL/min. To the granules, tannic acid and magnesium stearate were added in the respective amounts shown in Table 1. Thus prepared composition for tableting was pressed with a rotary tableting machine ("HT-EX12SS-U" manufactured by Hata Iron Works Co., Ltd.) at a pressure of 25 N to obtain a tablet with 11 mm in diameter and 500 mg in weight.

[Table 1]

|  | ingredient | amount (weight part) |
|---|---|---|
| **composition for granulation** | **matrix PVA** | **39.9** |
|  | **metformin** | **48.8** |
|  | **crystalline cellulose** | **10.3** |
|  | **binder PVA** | **1.0** |
| **added when tableting** | **tannic acid** | **Variable amount** |
|  | **magnesium stearate** | **1.0** |

The following two types of PVA were used for the matrix PVA-based resin.

PVA1: average polymerization degree 2500, saponification degree 88 mol%, alkali metal salt content 0.03% by weight, and average particle size 48 μm, 78 μm, or 97 μm
PVA2: average polymerization degree 600, saponification degree 88 mol%, alkali metal salt content 0.03% by weight, and average particle size 320 μm

Tablets No. 1 to No. 7 were manufactured with use of the matrix PVA-based resins shown in Table 2. Tablets No. 1 to No. 7 differed in a mixing ratio of tannic acid to the matrix PVA-based resin as shown in Table 2. Table 2 also shows the measurement results of hardness and release rate of the produced tablets.

The average polymerization degree of the PVA-based resin each contained in tablets Nos. 1 to 6 was 2454 according to the calculation by the equation shown below. While the average polymerization degree of the PVA-based resin contained in tablet No. 7 was 600.

$$\text{Average polymerization degree} = 2500 \times 39.9/40.9 + 600 \times 1/40.9 = 2454$$

[Table 2]

| Tablet No | matrix PVA | | amount of tannic acid | | | evaluation | | | |
|---|---|---|---|---|---|---|---|---|---|
| | average of polymerization degree | particle diameter ($\mu$m) | amount | per matrix PVA*1 | per metformin*2 | release rate (%) | | | hardness (N) |
| | | | | | | 1h | 3h | 10h | |
| 1 | 2500 | 48 | 4.0 | 0.1 | 0.08 | 40.5 | 67.4 | 100.0 | 123 |
| 2 | 2500 | 78 | 4.0 | 0.1 | 0.08 | 39.3 | 65.7 | 100.0 | 101 |
| 3 | 2500 | 97 | 4.0 | 0.1 | 0.08 | 39.8 | 65.5 | 100.0 | 96 |
| 4 | 2500 | 48 | 8.0 | 0.2 | 0.16 | 35.3 | 61.1 | 100.0 | 137 |
| 5 | 2500 | 48 | 12.0 | 0.3 | 0.25 | 38.7 | 66.4 | 100.0 | 151 |
| 6 | 2500 | 48 | 0 | 0 | 0 | 45.4 | 79.6 | 100.0 | 147 |
| 7 | 600 | 43 | 4.0 | 0.1 | 0.08 | 83.4 | 100.0 | 100.0 | 101 |
| *1 amount of tannic acid per 1 weight part of matrix PVA *2 amount of tannic acid per 1 weight part of metformin | | | | | | | | | |

A comparison between No. 1 and No. 6 shows that coexistence of tannic acid contributed to sustained-release of the tablet.
From Nos. 1, 4 and 5 differing only in the content of tannic acid, it can be understood that the hardness increases as the increase of tannic acid content.
From the comparison between No. 1 and No. 7, when a PVA having a low average polymerization degree like a binder PVA is used as a matrix polyvinyl alcohol-based resin, the resulting tablet is not considered to assure a desirable sustained-release performance.

INDUSTRIAL APPLICABILITY

Solid dosage form such as a tablet produced using a composition for solid dosage form of the disclosure have a sustained-release property and desirable hardness. Therefore, the composition for solid dosage form of the disclosure is useful because combining a variety of pharmaceutical ingredients requiring sustained-release with the composition of the disclosure would provide a desired sustained-release tablet containing the pharmaceutical ingredient.

Claims

1. A composition for sustained-release solid dosage form comprising

(A) a polyvinyl alcohol-based resin having an average saponification degree of 78 to 96 mol% and an average polymerization degree of 800 or more, the polyvinyl alcohol-based resin (A) being a powder having an average particle size of 40 to 200 $\mu$m measured by a laser diffraction method; and
(B) tannic acid as a polyhydric phenol compound.

2. The composition for sustained-release solid dosage form according to claim 1, wherein the content of the polyhydric phenol compound (B) is from 0.1 to 35 parts by weight with respect to 100 parts by weight of the polyvinyl alcohol-based resin (A).

3. The composition for sustained-release solid dosage form according to claim 1 or 2, wherein the polyvinyl alcohol-based resin (A) has a modification degree of less than 10 mol%.

4. The composition for sustained-release solid dosage from according to any one of claims 1 to 3, wherein the polyvinyl alcohol-based resin (A) is a mixture of a first polyvinyl alcohol-based resin having an average polymerization degree of 1500 or more to 3000 and a second polyvinyl alcohol-based resin having an average polymerization degree of 100 to less than 1500.

5. The composition for sustained-release solid dosage form according to any one of claims 1 to 4, further comprising (C) crystalline cellulose in an amount of 1 to 50 parts by weight with respect to 100 parts by weight of the polyvinyl alcohol-based resin (A).

6. A method for producing a sustained-release tablet, comprising

mixing tannic acid powder with a granule of a composition containing a pharmaceutical ingredient and a polyvinyl alcohol-based resin having an average saponification degree of 78 to 96 mol% and an average polymerization degree of 800 or more; and
tableting the mixture.

7. The method for producing a sustained-release tablet according to claim 6, wherein the polyvinyl alcohol-based resin has an average polymerization degree of 1500 to 3000 and is in form of powder having an average particle size of 40 to 200 $\mu$m measured by a laser diffraction method.

8. The method for producing a sustained-release tablet according to claim 6 or 7, wherein the granules are prepared by a fluid bed granulation process.

9. A use of a composition comprising

(A) a polyvinyl alcohol-based resin having an average saponification degree of 78 to 96 mol% and an average polymerization degree of 800 or more, the polyvinyl alcohol-based resin (A) being a powder having an average particle size of 40 to 200 $\mu$m measured by a laser diffraction method; and
(B) tannic acid as a polyhydric phenol compound;

for providing a tablet with sustained -release property.

10. A sustained-release tablet comprising a composition according to any one of claims 1 to 5, and a pharmaceutical ingredient.

11. A sustained-release tablet according to claim 10, wherein the pharmaceutical ingredient is a drug classified into Class I or III in Biopharmaceutical Classification System.


**Patentansprüche**

1. Eine Zusammensetzung für eine feste Darreichungsform mit verzögerter Freisetzung, umfassend

(A) ein Harz auf Polyvinylalkoholbasis mit einem mittleren Verseifungsgrad von 78 bis 96 mol-% und einem mittleren Polymerisationsgrad von 800 oder mehr, wobei das Harz auf Polyvinylalkoholbasis (A) ein Pulver mit einer mittleren Teilchengröße von 40 bis 200 $\mu$m, gemessen durch ein Laserbeugungsverfahren, ist; und
(B) Gerbsäure als mehrwertige Phenolverbindung.

2. Die Zusammensetzung für eine feste Darreichungsform mit verzögerter Freisetzung nach Anspruch 1, wobei der Gehalt der mehrwertigen Phenolverbindung (B) 0,1 bis 35 Gewichtsteile, bezogen auf 100 Gewichtsteile des Harzes auf Polyvinylalkoholbasis (A), beträgt.

3. Die Zusammensetzung für eine feste Darreichungsform mit verzögerter Freisetzung nach Anspruch 1 oder 2, wobei das Harz auf Polyvinylalkoholbasis (A) einen Modifikationsgrad von weniger als 10 mol-% aufweist.

4. Die Zusammensetzung für eine feste Darreichungsform mit verzögerter Freisetzung nach einem der Ansprüche 1 bis 3, wobei das Harz auf Polyvinylalkoholbasis (A) ein Gemisch eines ersten Harzes auf Polyvinylalkoholbasis mit einem mittleren Polymerisationsgrad von 1500 oder mehr bis 3000 und eines zweiten Harzes auf Polyvinylalkoholbasis mit einem mittleren Polymerisationsgrad von 100 bis weniger als 1500 ist.

5. Die Zusammensetzung für eine feste Darreichungsform mit verzögerter Freisetzung nach einem der Ansprüche 1 bis 4, ferner umfassend (C) kristalline Cellulose in einer Menge von 1 bis 50 Gewichtsteilen, bezogen auf 100 Gewichtsteile des Harzes auf Polyvinylalkoholbasis (A).

6. Ein Verfahren zur Herstellung einer Tablette mit verzögerter Freisetzung, umfassend Mischen von Gerbsäurepulver mit einem Granulat einer Zusammensetzung, die einen pharmazeutischen Inhaltsstoff und ein Harz auf Polyvinylalkoholbasis mit einem mittleren Verseifungsgrad von 78 bis 96 mol-% und einem mittleren Polymerisationsgrad von 800 oder mehr enthält; und
Tablettieren des Gemischs.

7. Das Verfahren zur Herstellung einer Tablette mit verzögerter Freisetzung nach Anspruch 6, wobei das Harz auf Polyvinylalkoholbasis einen mittleren Polymerisationsgrad von 1500 bis 3000 aufweist und in Form eines Pulvers mit einer mittleren Teilchengröße von 40 bis 200 $\mu$m, gemessen durch ein Laserbeugungsverfahren, vorliegt.

8. Das Verfahren zur Herstellung einer Tablette mit verzögerter Freisetzung nach Anspruch 6 oder 7, wobei das Granulat durch ein Fließbettgranulationsverfahren hergestellt wird.

9. Eine Verwendung einer Zusammensetzung, umfassend

(A) ein Harz auf Polyvinylalkoholbasis mit einem mittleren Verseifungsgrad von 78 bis 96 mol-% und einem mittleren Polymerisationsgrad von 800 oder mehr, wobei das Harz auf Polyvinylalkoholbasis (A) ein Pulver mit einer mittleren Teilchengröße von 40 bis 200 $\mu$m, gemessen durch ein Laserbeugungsverfahren, ist; und
(B) Gerbsäure als mehrwertige Phenolverbindung;

zur Bereitstellung einer Tablette mit verzögerter Freisetzungseigenschaft.

10. Eine Tablette mit verzögerter Freisetzung, umfassend eine Zusammensetzung nach einem der Ansprüche 1 bis 5 und einen pharmazeutischen Inhaltsstoff.

11. Eine Tablette mit verzögerter Freisetzung nach Anspruch 10, wobei der pharmazeutische Inhaltsstoff ein Arzneistoff ist, der in Klasse I oder III des Biopharmazeutischen Klassifizierungssystems eingestuft ist.

**Revendications**

1. Composition pour forme posologique solide à libération prolongée comprenant

(A) une résine à base de poly(alcool vinylique) ayant un degré de saponification moyen de 78 à 96 % en moles et un degré de polymérisation moyen de 800 ou plus, la résine à base de poly(alcool vinylique) (A) étant une poudre ayant une granulométrie moyenne de 40 à 200 $\mu$m, mesurée par une méthode de diffraction laser ; et
(B) de l'acide tannique en tant que composé phénolique polyvalent.

2. Composition pour forme posologique solide à libération prolongée selon la revendication 1, dans laquelle la teneur en le composé phénolique polyvalent (B) est de 0,1 à 35 parties en poids pour 100 parties en poids de la résine à base de poly(alcool vinylique) (A).

3. Composition pour forme posologique solide à libération prolongée selon la revendication 1 ou 2, dans laquelle la résine à base de poly(alcool vinylique) (A) a un degré de modification inférieur à 10 % en moles.

4. Composition pour forme posologique solide à libération prolongée selon l'une quelconque des revendications 1 à 3, dans laquelle la résine à base de poly(alcool vinylique) (A) est un mélange d'une première résine à base de poly(alcool vinylique) ayant un degré moyen de polymérisation de 1 500 ou plus à 3 000 et d'une deuxième résine à base de poly(alcool vinylique) ayant un degré moyen de polymérisation de 100 à moins de 1 500.

**5.** Composition pour forme posologique solide à libération prolongée selon l'une quelconque des revendications 1 à 4, comprenant en outre une cellulose cristalline (C) en une quantité de 1 à 50 parties en poids pour 100 parties en poids de la résine à base de poly(alcool vinylique) (A).

**6.** Méthode pour produire un comprimé à libération prolongée, comprenant le mélange de poudre d'acide tannique avec un granulé d'une composition contenant un ingrédient pharmaceutique et une résine à base de poly(alcool vinylique) ayant un degré de saponification moyen de 78 à 96 % en moles et un degré de polymérisation moyen de 800 ou plus ; et

la mise en comprimé du mélange.

**7.** Méthode pour produire un comprimé à libération prolongée selon la revendication 6, dans laquelle la résine à base de poly(alcool vinylique) a un degré de polymérisation moyen de 1 500 à 3 000 et est sous la forme d'une poudre ayant une granulométrie moyenne de 40 à 200 $\mu$m, mesurée par une méthode de diffraction laser.

**8.** Méthode pour produire un comprimé à libération prolongée selon la revendication 6, dans laquelle les granulés sont préparés par un procédé de granulation en lit fluidisé.

**9.** Utilisation d'une composition comprenant

(A) une résine à base de poly(alcool vinylique) ayant un degré de saponification moyen de 78 à 96 % en moles et un degré de polymérisation moyen de 800 ou plus, la résine à base de poly(alcool vinylique) (A) étant une poudre ayant une granulométrie moyenne de 40 à 200 $\mu$m, mesurée par une méthode de diffraction laser ; et
(B) de l'acide tannique en tant que composé phénolique polyvalent,

pour former un comprimé ayant une propriété de libération prolongée.

**10.** Comprimé à libération prolongée comprenant une composition selon l'une quelconque des revendications 1 à 5 et un ingrédient pharmaceutique.

**11.** Comprimé à libération prolongée selon la revendication 10, dans lequel l'ingrédient pharmaceutique est un médicament classé dans la Classe 1 ou III du Système de Classification Biopharmaceutique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6657949 B
- JP 2020152658 A
- JP 2013241341 A
- JP 2014224086 A
- WO 2015115453 A

**Non-patent literature cited in the description**

- **IKEUCHI-TAKAHASHI et al.** *Biol. Pharm. Bull*, 2016, vol. 39, 1514-1522
- *Biol. Pharm. Bull.*, 2016, vol. 39, 1514-1522